# EUROPEAN PATENT SPECIFICATION

(11) **EP 0 452 476 B1**
(45) Date of publication and mention of the grant of the patent: **31.08.1994**
(21) Application number: 90917660.4
(22) Date of filing: 09.11.1990
(51) Int. Cl.: A61M 39/00

(54) **BAYONET LOCK CANNULA FOR PRE-SLIT Y-SITE**
KANÜLE MIT BAJONETTVERSCHLUSS FÜR VORGESCHLITZTES Y-FÖRMIGES ANSATZSTÜCK
CANULE A VERROUILLAGE A BAIONNETTE POUR EMBOUT EN Y PREFENDU

(30) Priority: 09.11.1989 US 435638
(43) Date of publication of application: 23.10.1991
(73) Proprietor: BAXTER INTERNATIONAL INC., Deerfield, IL 60015 (US)
(72) Inventor: DESECKI, Vincent, C., Ingleside, IL 60041 (US); DUDAR, Thomas, E., Palatine, IL 60067 (US); FINLEY, Michael, J., Park City, IL 60085 (US)
(74) Representative: MacGregor, Gordon
(86) International application number: US9006572
(87) International publication number: WO9107206

(56) References cited:
- EP-A- 0 319 764
- EP-A- 0 367 549
- WO-A-89/06553
- US-A- 4 511 359
- US-A- 4 796 615

## Description

The present invention relates to an assembly of a Y-type fluid flow connector, having an injection site, with a cannula insertion member having a cannula for insertion through the injection site.

One injection site usable with a piercing cannula is disclosed in U. S. Patent No. 4,412,573.

The pointed cannula can be forced through the septum into fluid flow communication with the flow path in the housing. Known injection sites usable with a piercing cannula can be physically damaged by repetitive piercing caused by the sharp cannula. This damage, known as coring or laceration, can result in subsequent leakage.

Due to problems associated with infectious agents, personnel using such pointed cannulae do so with great care. Notwithstanding careful and prudent practice, from time to time, accidents do occur and individuals using such pointed cannulae jab themselves.

Injection sites usable with a blunt cannula are also known. For example, U. S. Patent No. 4,197,848 discloses one such injection site. That infection site is a relatively low pressure device having a relatively thin, molded, sealing member. The sealing member has an opening therethrough.

A blunt cannula can be forced through the sealing member placing the cannula into fluid flow communication with a fluid flow pathway in the injection site.

Injection sites of the type noted above usable with a blunt cannula have the advantage that the blunt cannula will not pierce the skin of a user. On the other hand, it is important that the pre-slit injection site reseal with enough force that fluids do not ooze therefrom and that airborne particulate matter, bacterial or viral matter do not enter therethrough.

WO-A-86/03416 describes, in relation to Figs 16 and 17, a cannula insertion member having a cannula insertable through an injection site in a Y-type connector. The device has a shield around the cannula, the shield engaging with the injection site housing. The shield extends beyond the arm of the Y-site and has a slot which receives the arm to permit such extension.

The precharacterising part of Claim 1 is based on this document.

The distinguishing features of the invention are set out in the characterising part of Claim 1 and include the shield having an arcuate arm extending generally circumferentially about the distal end region of the cannula, the shield and connector being relatively rotatable to engage the arcuate arm beneath the arm of the connector in bayonet fashion to lock the cannula insertion member on the connector and the cannula having a tapered distal end region leading to a blunt distal end.

EP-A-0319764 discloses in Fig 12 a Y-connector with an injection site in its arm. A sleeve is mounted on the arm for receiving a cannula insertion member, which has screw-engagement in the sleeve.

The document also discloses an injection site provided externally with a pin and a cannula insertion member having a sleeve provided with an L-shaped slot, the pin being engageable in bayonet fashion in the slot.

US-A-4511359 also discloses a sterile connection device utilising a pin on one member and an L-shaped slot in the other.

Neither of these two documents discloses a cannula with a tapered distal end region leading to a blunt distal end. This feature is not disclosed either in EP-A-036756, which is relevant with respect to Article 54(3), but not Article 56 EPC.

Reference is also made to WO-A-89/06553, which discloses a preslit injection site and tapered, blunt cannula.

### Brief Description of the Drawings

Figure 1 is a side elevational view, partly in section, of a prior art pre-slit injection site and an associated blunt cannula;
Figure 2 is an overall view of a container, an associated solution administration set and a pre-slit injection Y-site in accordance with the present invention;
Figure 3 is a side view of the Y-site connector in section carrying a pre-slit injection site in accordance with the present invention;
Figure 4 is an enlarged fragmentary side view in section of a coupling member carrying a pre-slit injection site where the slit extends only partway through the septum;
Figure 5 is a perspective view of a pre-slit Y-site and bayonet lock cannula bounded to tubing.
Figure 6 is an exploded perspective view of a pre-slit Y-site and bayonet lock cannula with a luer lock connection to tubing.
Figure 7 is a perspective view of a bayonet lock cannula having two bayonet locks.
Figure 8 is a side view in section of the bayonet lock cannula.
Figure 9 is a perspective view with a partial sectional of the pre-slit Y-site with bayonet lock cannula.
Figure 10 is a side view of the pre-slit Y-site with bayonet lock cannula.
Figure 11 is a top view of the bayonet lock cannula with a luer lock connection.
Figure 12 is a side sectional view of the pre-slit Y-site with bayonet lock cannula in the locked position.
Figure 13 is a sectional view of a first embodiment of a blunt cannula.
Figure 14 is a top view of the blunt cannula of Figure 13.
Figure 15 is a second embodiment of a blunt cannula.
Figure 16 is a top view of the blunt cannula of Figure 15.
Figure 17 is a third embodiment of a blunt cannula.
Figure 18 is a schematic exploded side view of the pre-slit Y-site and bayonet lock cannula with zones of interference marked.
Figure 19 is a step in the method of making a pre-slit injection site in accordance with the present invention.
Figure 20 is another step in the method of making a pre-slit injection site in accordance with the present invention.
Figure 21 is an initial phase of a final step in making a pre-slit injection site in accordance with the present invention.
Figure 22 is an intermediate phase of the final step in a method of making a pre-slit injection site in accordance with the present invention.
Figured 23 is a final phase of the final step in a method of making a pre-slit injection site in accordance with the present invention.
Figure 24 illustrates an initial phase in an alternate step of making a pre-slit injection site in accordance with the present invention.
Figure 25 illustrates a final phase of the alternate step in a method of making an injection site in accordance with the present invention.
Figure 26 illustrates yet another alternate step in a method of making a pre-slit injection site in accordance with the present invention.
Figure 27 is an enlarged, fragmentary cross-sectional view of another embodiment of an injection site in accordance with the present invention.
Figure 28 is a cross-section view taken generally along the plane 29-29 in Figure 27.

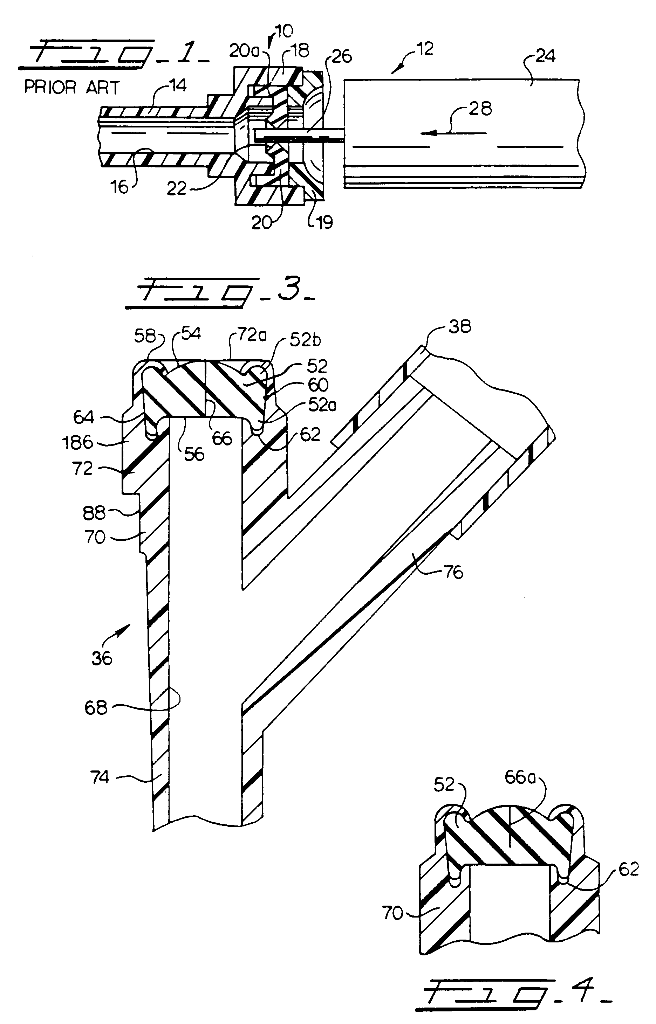

A prior art pre-slit injection site 10 and associated blunt cannula 12 are illustrated in Figure 1. The prior art injection site 10 has a cylindrical housing 14 with a fluid flow path 16 therethrough. A first end 18 of the housing 14 is closed with a relatively thin disc-shaped resealable member 20. The member 20 has a resealable opening 22 therein.

The member 20 is a molded septum with an integrally formed skirt 20a. The skirt 20a is oriented generally perpendicular to the portion of the septum with the opening 22.

The cannula 12 includes a body portion 24 which carries at a first end a hollow, cylindrical, blunt piercing member 26. As the cannula 12 is moved in a direction 28 toward the first end 18 of the injection site 10, the member 26 slidably engages the opening 22. The sealing member 20 is then deformed adjacent the opening 22 and the member 26 extends into the flow path 16. A fluid flow path through the cannula 12 will then be in fluid flow communication with the flow path 16 via the hollow piercing member 26.

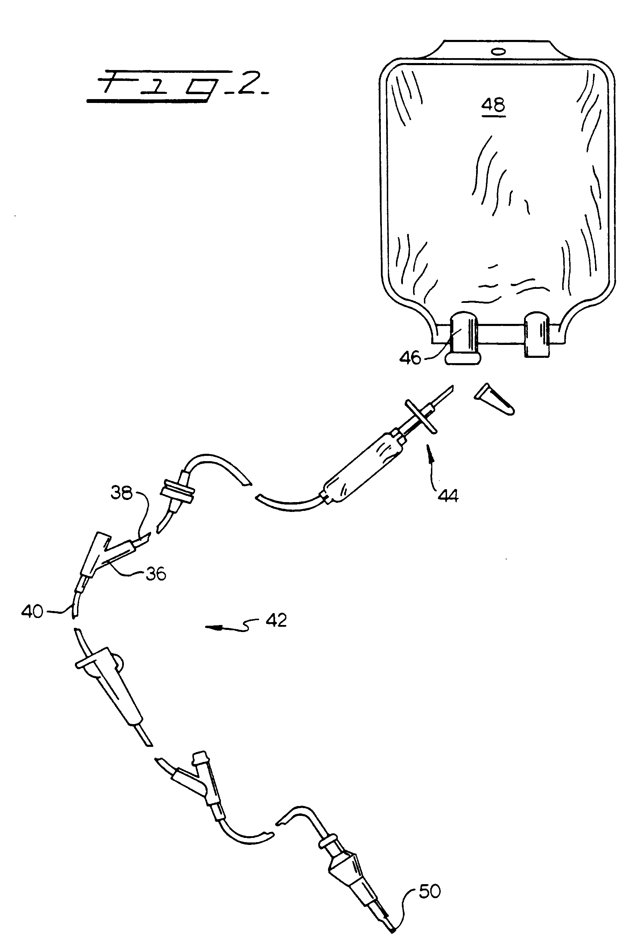

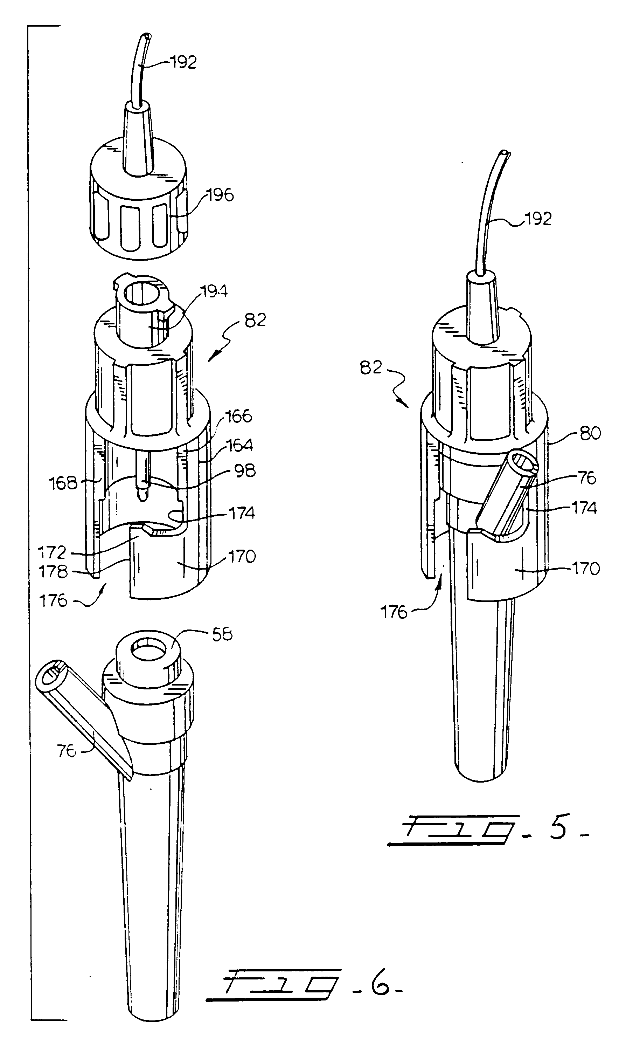

Figures 2 and 3 illustrates a pre-slit septum 52 in a Y-site connector or Y-site 36 which is connected to first and second pieces of tubing, 38 and 40, respectively. Figure 2 shows a fluid administration set 42 of a conventional variety except that it includes as a component the pre-slit Y-site 36. The set 42 includes a spike connection 44 at a first end designed to pierce the port 46 of the container 48. The set 42 may also include other components such as adjustable clamps or filters. At the second end of the set 42 is a piercing member 50 which connects the fluid flow to the patient, such as through a vein in the hand of the patient.

As shown in Figure 3, Y-site 36 is formed with a cylindrical housing 70 having a first end 72, and a second end 74 and one arm 76. As discussed above, the second end 74 is bounded to second tubing 40 and the arm 76 is bounded to first tubing 38. The primary flow of fluid is from container 48 through arm 76 and second end 74 to the patient. The secondary fluid flow from a second container, syringe or other medical device (not shown) is through first end 72 and second end 74 to the patient. The primary fluid and the secondary fluid are mixed in the second end 74 of the Y-site housing and in second tubing 40.

Figure 3 illustrates, in section, further details of the Y-site 36. A resealable septum 52 is carried by the first end 72 of the housing 70. The septum 52 includes first and second spaced apart surfaces 54 and 56 respectively. The surface 54 has been forced into a dome-like shape by annular, U-shaped, swaged end members 58 carried by the first end 72. The dome-like shape of the surface 54 can extend beyond a surface 72a of the first end 72. This facilitates cleaning the surface 54.

The septum 52 has a generally cylindrical shape. The septum 52 can be formed of a latex or synthetic rubber material. Alternately, the septum can be formed of a thermosplastic elastomer. The material used for the septum 52 should be non-toxic and sterilizable such as by means of radiation, steam or ethylene oxide.

Because the septum 52 is generally cylindrical in shape, it can be die-cut from a sheet, cut from an extruded rod or molded. The septum 52 can have an exemplary diameter on the order of 7.6 mm (.30 inches). The height of the septum 52 can be, for example, on the order of 3.2 mm (.125 inches).

The first end 72 is also formed with a tapered interior surface 60 which terminates in an annular channel 62. The tapered interior surface 60 has a taper in a range of 5 degrees to 20 degrees. Preferably, the taper will be on the order of 7 degrees. With the indicated size of the above noted exemplary septum 52 and a 7 degree taper, diametric resealing compression of the septum 52 adjacent the channel 62 is on the order of 10%.

The channel 62 is bounded in part by a septum supporting ridge. The channel 62 can typically have a depth in a range of 1.27 mm - 1.78 mm (.050-.070 inches).

A peripheral surface 64 of the septum 52 slidably engages the tapered interior surface 60 as the septum 52 slides into the first end 72. The annular channel 62 which underlies the interior peripheral surface 64 of the septum 52 is provided to permit the septum 52 to deform when a blunt cannula is inserted through an opening 66 therein.

The swaged end members 58 apply axial forces to the septum 52 thereby creating the domed exterior peripheral surface 54. The axial forces applied by the end members 58 slightly deform the regions 52a and 52b. The tapered internal surface 60 applies radially directed forces to the septum 52, thereby forcing the opening 66 into a resealed condition.

In an alternate embodiment, the surface 54 could be formed as a flat, as opposed to a domed, surface.

Once the Y-site 36 is lockingly engaged with the bayonet lock cannula 82, as will be discussed later, a sealed system is formed through which fluids can be infused into the Y-site 36. The resealable septum 52 closes the fluid flow path through Y-site 36 when the bayonet lock cannula 82 is disengaged.

As discussed in EP-A-0 354 947, the injection site and blunt cannula may be adapted for use with a variety of medical instruments, including, but not limited to a syringe with luer locking connection, a blunt cannula with deflecting lever locking members, or a spike connection for an IV solution bag.

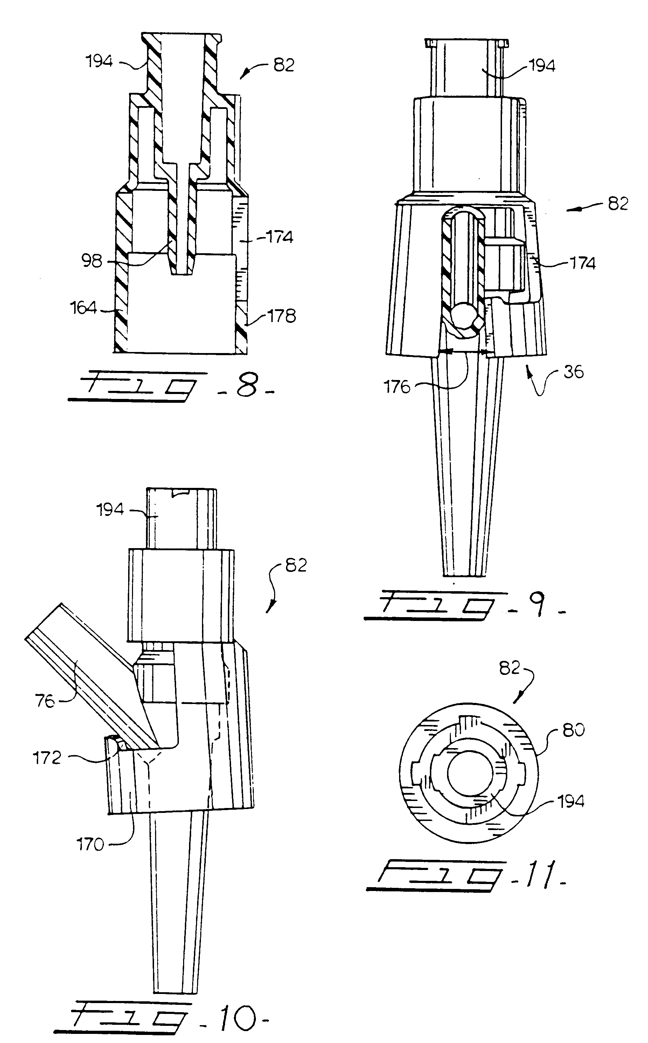

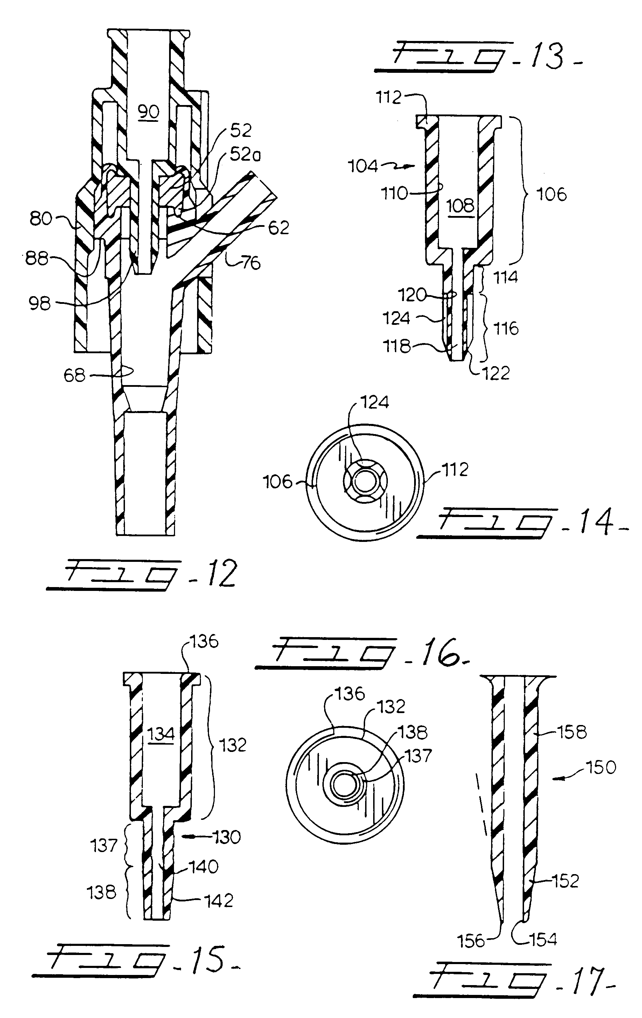

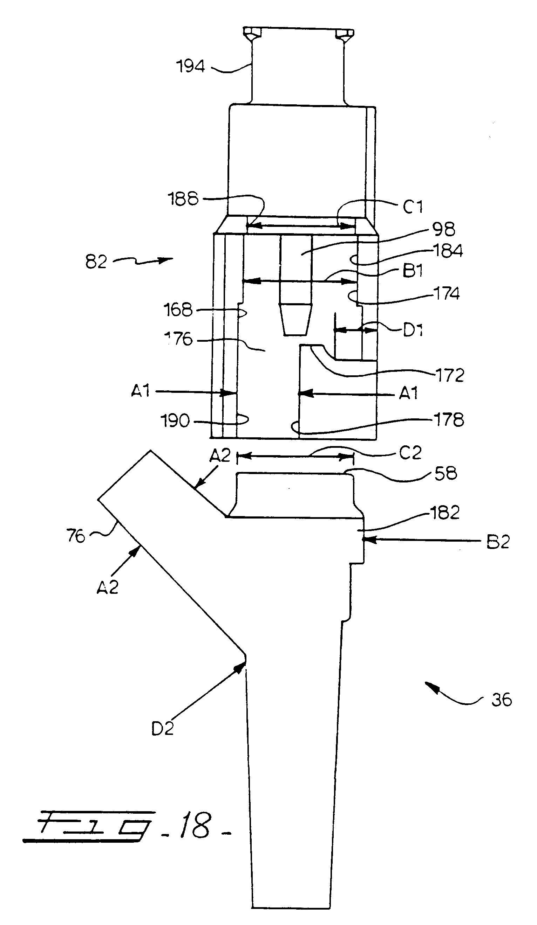

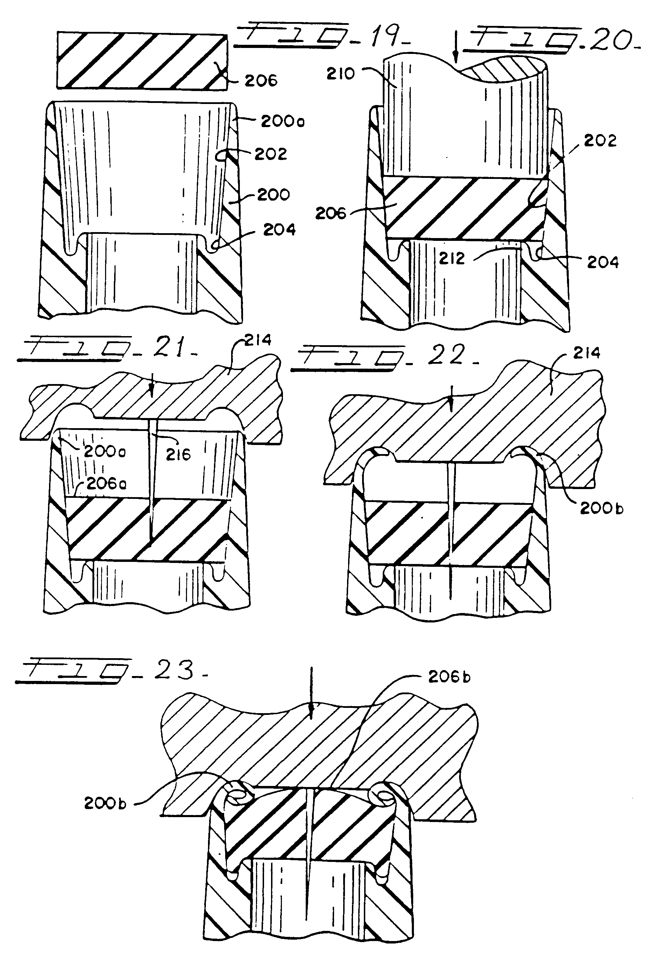

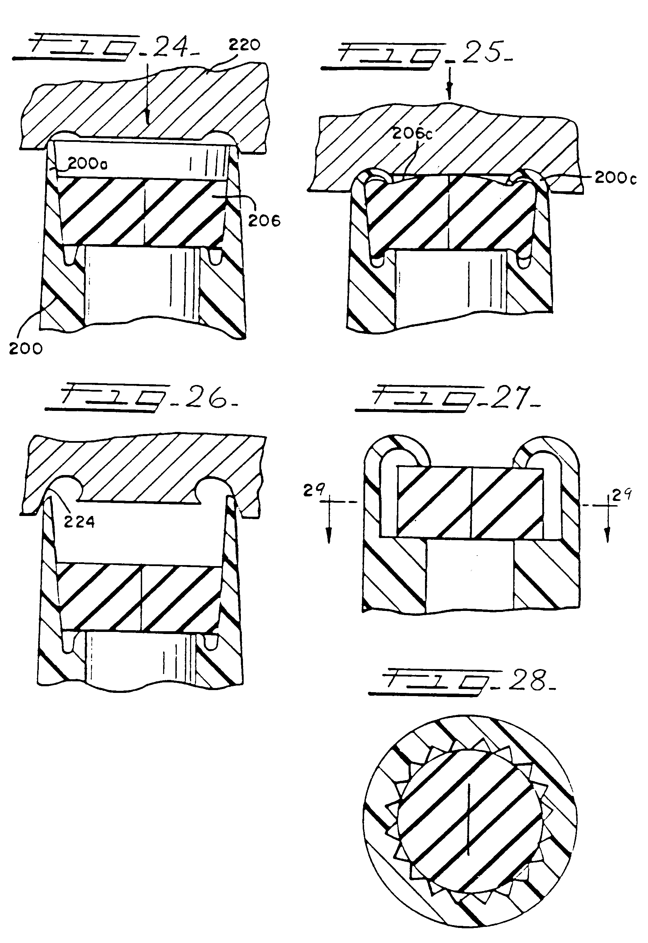

In addition to being used with the bayonet locking cannula 82 of Figures 7-12; the Y-site of the present invention may be also used with a variety of other cannulas, including the cannulas shown in Figures 13 through 17.

The cannula shown in Figures 13-17 may be incorporated into a bayonet locking cannula to achieve a locked blunt cannula/Y-site connection as will be described later.

Figures 13 and 14 illustrate a tapered cannula structure 104 which is a first embodiment that can be used with the Y-site 36 and incorporated into the bayonet locking cannula 82. The cannula includes a proximal end 106 with an interior region 108. The interior region 108 is in part bounded by an internal peripheral wall 110 which is formed with a standard luer taper. The tapered cannula 104 can be formed with a luer-type coupling flange 112 at the proximal end so as to be releasably connected to another medical device, such as a syringe.

Extending from the proximal end 106 is a cylindrical tube having a cylindrical mid-region 114 and a distal end member 116. The distal end member 116 has a generally elongated, cylindrical shape with an exterior side wall 120. A centrally located, cylindrical, internal fluid flow path 118 extends through distal end member 116 and mid-region 114 and fluid flow communication with the interior region 108. The distal end of end member 116 has a tapered exterior surface 122. The tapered exterior surface 122 minimizes insertion force of the cannula 104 is being forced through a slit of the septum, such as the slit 66 of septum 52. The angle of taper of the surface 122 is preferably in the range between 1 to 15 degrees.

The distal end member 116 is also provided with a plurality of circumferentially spaced elongated grooves 124. The grooves 124 in the exterior wall of the distal end member 116 extend longitudinally of the cannula and decrease the surface area contact at the cannula/septum interface during insertion of the cannula and to the Y-site 36. This reduced exterior contact surface area decreases the frictional component of the insertion force.

In one embodiment, the tapered blunt cannula 104 may have an overall insertion length, corresponding to the axial length of mid-region 114 and end member 116, on the order of 9.52 mm (0.375 inches).

An alternate cannula structure, identified generally as numeral 130, is shown in Figures 15 and 16. The cannula 130 includes a proximal end 132 defining an interior region 134 and having a luer-type flange 136 for connection to a suitable mating engaging surface.

A generally cylindrical mid-region 137 extends between the proximal end 132 and a distal end region 138. The embodiment of the cannula 130 minimizes kick back or recoil due to the provision of substantially cylindrical mid-region 137. This design also increases tug resistance to reduce inadvertent withdrawal.

A generally cylindrical internal flow channel 140 extends through the end region 138 and mid-region 137 in communication with the interior region 134 of the proximal end region 132. The end region 138 is provided with a tapered surface 142. The design permits of a very small taper to minimize the insertion force. Further the design permits the cannula 130 to be constructed with a small tip diameter, small tapered angle and small cannula diameter so as to reduce the peak insertion force.

Still another embodiment is illustrated in Figure 17 which shows a blunt tapered cannula insertion member 150 for insertion into the pre-slit injection Y-site. The cannula 150 has a distal end region 152 with a tapered exterior surface in which the preferred embodiment is an approximately 8°. The defined aperture 154 for fluid flow is disposed at the end region 156 of the distal end region 152. The end 156 includes a radiused tip defined by a radius of approximately 0.25 mm (0.01 inch). The radiused tip reduces insertion force, assists in locating the slit in the injection site and in addition has the practical advantage of facilitating complete filling of the cannula mold cavity.

The tapered surface of the distal end region 152 has an axial length of approximately 2.5 mm (0.10 inches) in the preferred embodiment. Adjacent to the tapered distal end region is a generally cylindrical region 158 for entering into the injection site behind the distal end region 152, thereby reducing kick back during insertion. The generally cylindrical region 158 has a small draft angle such as about one-half degree.

The force required to insert any of the above-discussed embodiments of a blunt tapered cannula into the septum of the injection site depends upon a number of factors: friction at the cannula/septum interface, cannula diameter, cannula tapered angle, and degree of septum compression. The cannula/septum interface friction is, in turn, dependent upon lubrication, if any, material properties and surface finish. It will be understood that the friction at the cannula/septum interface can be reduced by providing a smoother surface finish on the cannula (e.g., by sandblasting the cannula exterior surface) or by molding the cannula so as to produce a matte finish. Conventional lubricants can also be used to further reduce friction and thereby lower the insertion force required.

The deformation of the septum 52 also facilitates insertion of the piercing member 98 through the slit or opening 66. Subsequent to the piercing member 98 slidably engaging the Y-type injection site or Y-site 36, the interior region of the tubing 192 to which the bayonet locking cannula 82 is connected is in fluid flow communication with the flow path 68 of the Y-site 36 via the flow path of the blunt piercing member 98.

In this engagement condition, the septum 52 seals completely around the piercing member 98. Hence, exterior gases, liquids or airborne matter will be excluded from the flow path 68.

Subsequent to infusing fluid from the bayonet locking cannula 82 into the fluid flow pathway 68, hence to the patient, the bayonet locking cannula 82 with lockingly engaged shielded cannula can be slidably withdrawn from the injection site 36. Subsequent to this withdrawal, the septum 52 reseals the opening or slit 66 therein.

The opening 66 will repeatedly reseal, when the piercing member 98 is removed, provided that the pressure (in the septum 52) created by interaction of the septum material properties and compression supplied by the housing exceeds the pressure challenge of the fluid contained within. Blunt cannulae do not haphazardly core, lacerate, or otherwise damage the sealing interface of slit 66 as conventional needles do, thereby allowing repeatable resealability. However, septum material properties, thickness, and compression allow resealability for a finite number of conventional needle insertions. The Y-site 36 then returns to its pre-infusion, sealed condition.

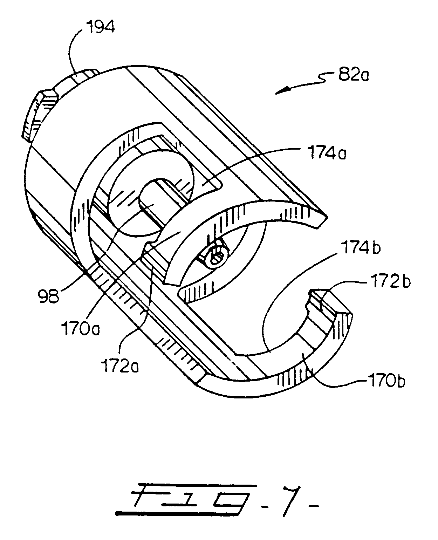

As an alternate to forming the slit 66 completely through the septum 52, as illustrated in Figure 4 a slit 66a can be formed only partly through the septum 52. Such a structure has the further advantage that until used for the first time the septum 52 is completely sealed.

The septum 52 can be formed in two parts. One part can have a slit, such as the slit 66 extending entirely therethrough. A second part can be formed without a slit. These two parts can be located adjacent one another in first end 72 of the injection site.

The slit 66a may be longer on the top of the septum than the bottom. This feature aids blunt cannula alignment with the slit upon insertion, and aids resealability by minimizing the critical slit sealing interface area.

The slit 66 could have a length with a range on the order of 0.76 mm to 3.8 mm (.03 to .150 inches). Preferably, a slit length in the order of 1.78 mm (.07 inches) will be used in combination with a blunt cannula having a diameter on the order of 2.5 mm (.1 inches).

When initially used, the blunt cannula piercing member 98 will be forced through the slit 66a. The lower peripheral surface of the septum 52 will then be punctured, providing access for the blunt cannula piercing member 98 into the fluid flow pathway 68.

Figures 19-23 disclose a method of making a pre-slit injection site in accordance with the present invention. In a first step, a housing 200 is provided. The housing 200 has an interior tapered surface 202 at a first end 200a thereof. The interior peripheral surface terminates in an annular channel 204. A cylindrical septum 206 can be provided adjacent the end 200a.

In a second step, the septum 206 can be forced into the end 200a of the housing 200 and slightly deformed by the tapered peripheral surface 202 using an axially moving die 210. When positioned by the die 210, the septum 206 is located adjacent an internal annular ring 212 which bounds the annular channel 204.

In a third step, a second die 214 can be utilized to swag the end 200a into spiral-shaped, spring-like members 200b which apply axially directed forces against an exterior peripheral surface 206a of the septum 206. The axially directed forces form the flat surface 206a into a domed exterior peripheral surface 206b as illustrated in Figure 23.

Simultaneously, with swaging the end members 200a so as to lock the septum 206 into the housing 200 and to form the domed exterior peripheral surface 206b, a knife 216 can be utilized to form a slit in the septum 206. Alternatively, the slit may be cut by a separate die in a separate step. If the septum 206 is formed as an extrusion, the slit can be created during the extrusion process. If the septum 206 is formed by stamping from a rubber sheet, the slit can be cut during the stamping process. If the septum 206 is formed by compression molding, the slit can be cut during the trimming process.

In order to extrude the slit into rod, a flat pin extrusion bushing can be used. A trailing ribbon may be attached to the bushing. The ribbon would prevent curing material across the slit. The ribbon or wire could be placed in the rod core and later stripped out leaving a slit. An inert substance, such as silicone oil, could be coextruded in the center of the rod to prevent curing across the slit and provide lubrication and a visible target for cannula insertion.

Figures 24 and 25 illustrate alternate swaging steps wherein a die 220 moving axially toward the housing 200 swages the end region 200a so as to form an annular U-shaped region 200c and the exterior domed peripheral surface 206c.

The dies 214 or 220 can be formed with various alternate shaped swaging surfaces 224, as illustrated in Figure 26, depending on the precise shape of the end swage which is desired.

The injection site configuration need not be limited to the configurations depicted in the figures. Rather, several configurations could be constructed. Any such configuration would provide a flexible pre-slit sealing member captured in a housing which provides compression to create a seal against pressure and a void region accessible to the sealing member material only when displaced by a blunt cannula piercing member. One such possible configuration is depicted in Figures 27 and 28.

In the embodiments of the cannulae described herein, the mid-region and the tapered distal end region may be alternatively characterized as together forming at least one tube defining a fluid flow path therein with the tube having a distal end region for penetrating the injection site.

In preferred contemplated embodiments, the exterior surface of the distal end region may have a taper angle as small as between one and fifteen degrees.

As shown in Figure 5, the bayonet locking blunt cannula 82 includes a cylindrical hollow protective shield 80 which surrounds a centrally located hollow, elongated cylindrical blunt piercing member 98 and extends beyond the blunt distal end of the cannula. The cylindrical blunt piercing member 98 has a total length on the order of 3 times the thickness of the septum 52 in order to ensure complete penetration. The cylindrical blunt piercing member 98 has a diameter on the order of 1/3 the diameter of the septum 52. The blunt piercing member 98 can slidably engage the septum 52 and extend through the preformed opening 66. As illustrated in Figure 12, when the piercing member 98 slidably engages and pierces the septum 52, the region 52a deforms by expanding into and filling, at least in part, the annular channel 62.

The bayonet locking cannula 82 shown in Figure 5 includes a blunt cannula similar to that shown, for example, in Figure 16 in addition to a protective shield 80. Protective shield 80 includes an arcuate wall 164 that extends approximately 180-200° from side 166 to flat side 168. The arcuate wall 164 provides substantial shielding to the blunt piercing member 98 to maintaining the piercing member 98 in an aseptic condition by preventing touch contamination prior to its being inserted into the pre-slit septum 52.

Protective shield 80 also includes a bayonet arm 170 which ends in a bayonet barb 172 to create an opening or bayonet lock 174. As the bayonet locking cannula 82 is pushed onto the Y-site 36, Y-site arm 76 passes through the gap 176 between the end 178 of the bayonet arm and side 168. When the bayonet cannula 82 is pushed as far as possible onto the Y-site 36, then the Y-site can be rotated with respect to the bayonet lock, causing the side arm 76 to pass over the bayonet barb 172 and into the bayonet lock 174.

Because the Y-site 36 is part of an administration set 42 which provides fluid to the patient for a relatively long period of time, it is important that leaks be reduced. Leaks may occur at the Y-site if the blunt piercing member is misaligned with the Y-site septum 52 by more than acceptable tolerance. Such leaks are quite undesirable because of the medical implications.

The bayonet lock cannula 82 and the Y-site 36 have three zones of docking to provide for correct alignment. These zones of interference are indicated on Figure 18.

The first zone of interference is indicated by A1 and A2 on Figure 18. As the bayonet lock cannula 82 is moved onto the Y-site 36, Y-site arm 76 moves through gap 176 between the end 178 of bayonet arm and flat side 168. Gap 176 is smaller than the diameter of diameter of Y-site arm 76. For example, the gap 176 may be 5.0 mm (.200") while the diameter Y-site arm is 5.59 mm (.220") to create a positive interference of 0.25 mm (.010") per side.

The second zone of interference is indicated by B1 and B2 on Figure 18. After the piercing member 98 begins to penetrate the opening 66 of septum 52, the housing shoulder 182 of the Y-site 36 and the first interior mid-region 184 of protective shield 80 engage to create additional interference and directional guidance between the two components. The housing shoulder 182 and the first interior mid-region 184 should have a positive interference of, for example, 0.025 mm (.001") per side or 0.064 mm (.0025") per side. There should be a maximum positive interference between these two areas of 0.102 mm (.004").

The third zone of interference between the Y-site 36 and the bayonet lock cannula 82 is zone C, indicated by C1 and C2 on Figure 18. As the bayonet lock cannula 82 continues to move onto Y-site 36, the swage member 58 holding the septum 52 in compression on Y-site 36 engages the second interior mid-region of protective shield 80. The second interior mid-region 188 has a diameter that is smaller than the first interior mid-region 184 which has a diameter that is smaller than the diameter of the outer interior region 190. As the swage member 58 contacts the second interior mid-region 188, the injection site is now docked and ready for engagement within the bayonet lock 174.

The Y-site 36 is engaged by rotating the Y-site arm 76 past the bayonet barb 172 into the bayonet lock 174 thereby engaging a mechanical lock as indicated by D1 and D2. The dimensions of the Y-site 36 and bayonet lock cannula 82 are such that when the septum 52 is higher than the swage member 58 the septum 52 must be compressed a slight amount against the cannula 82 to rotate Y-site 76 arm past the bayonet barb 172. When the Y-site arm 76 is properly positioned within bayonet lock 174, the septum 52 is still compressed a slight amount. The compression of the septum 52 and the drag from the cannula 82 against the septum 52 require a predetermined torque to engage the Y-site 36 and to disengage the Y-site 76 arm from the bayonet lock 174.

The dimensions of the bayonet lock cannula 82 and Y-site 36 used in creating the zones of interference must be carefully calculated so that the torque is not so large that in engaging or disengaging the Y-site 36 from the bayonet lock 174, components adjacent to the Y-site 36 and bayonet lock cannula 174 are disconnected. Figure 5 shows a bayonet lock cannula 82 bonded to tubing 192 while Figure 6 shows a bayonet lock cannula 82 having a luer fitting 194 which mates with a luer cap 196 which is bonded to tubing 192. American National Standards Institute standards require that a locking luer fitting must not disconnect until 3 inch ounces of force (.021 Newton-metres) is exerted on it. If the connection or disconnection of the Y-site arm 76 from the bayonet lock 174 requires more force than this, the luer cap 196 may be accidentally disconnected from the luer fitting 194.

An alternative embodiment of the bayonet lock cannula 82 is shown in Figure 7 and generally identified as 82a. Bayonet lock cannula 82a includes two bayonet arms 170a-170b, two bayonet barbs 172a-172b, and two bayonet locks 174a-174b which mate with the two arms of a T-type connector with injection site or T-site (not shown).

## Claims

1. An assembly of a Y-type or T-type connector with a cannula insertion member, wherein the connector has a housing (70) provided at one end with a pre-slit injection site (52) and at the opposite end with a fluid outlet (74), and a tubular arm (76) projecting from the housing, and the cannula insertion member (82) comprises a cannula (98,104) defining a fluid flow path therein, the cannula having a distal end region for penetrating the injection site (52) and having at least one aperture in the distal end region through which fluid can flow to or from the fluid flow path, and a protective shield (80) at least partially surrounding and being spaced apart at a predetermined distance from the cannula, the shield (80) having an arcuate wall (164) partially surrounding and being spaced apart from the cannula (98,104), the arcuate wall defining an open side (174), the shield being engageable over the connector housing (70) as the cannula (98,104) is inserted in the injection site (52), with the connector arm (76) received in the open side (174), characterised by at least one arcuate arm (170) extending from said arcuate wall (164) across a portion of said open side (174), said arm extending generally circumferentially about the distal end region of the cannula (98), the shield (80) and the connector being relatively rotatable to engage the shield arcuate arm (170) beneath the connector arm (76) in bayonet fashion to lock the cannula insertion member on the connector, and the cannula having a tapered distal end region leading to a blunt distal end.

2. An assembly according to Claim 1, wherein the shield arm (170) has a barb (172), over which the connector arm (76) passes during said relative rotation.

3. An assembly according to Claim 1 or 2, wherein the protective shield (80) and the connector have at least one zone of interference (A1,A2;B1,B2;C1,C2;D1,D2) establishing alignment of the cannula insertion member (82) with the injection site (52).

4. An assembly according to Claim 3, including a zone of interference (A1,A2) between the connector arm (76) and the walls (168,178) defining a gap (176) between the shield arcuate arm (170).

5. An assembly according to Claim 3 or 4, including a zone of interference (B1,B2;C1,C2) between the interior of the shield (80) and the connector housing (70).

6. An assembly according to Claim 5, comprising spaced zones of interference (B1,B2;C1,C2) between the interior of the shield (80) and the connector housing (70).

7. An assembly according to any preceding claim wherein the shield (80) extends beyond the blunt distal end of the cannula (98,104).

8. An assembly according to any preceding claim, wherein the cannula (104) has a distal end portion (116) provided on its exterior surface with a plurality of circumferentially spaced elongate grooves (124) extending longitudinally of the cannula.

9. An assembly according to any preceding claim, wherein the cannula (98,104) has a matte finish to reduce friction at the cannula/septum interface.

10. An assembly according to any preceding claim, wherein the connector is a T-connector and the shield has a further arcuate arm (170b) for engaging an arm of the connector in bayonet fashion.

11. An assembly according to Claim 10, wherein said further arcuate arm (170b) has a barb (172b).

## Patentansprüche

1. Anordnung eines Y- oder T-Verbinders mit einem Kanüleneinführelement, wobei der Verbinder ein Gehäuse (70), das an einem Ende mit einer vorgeschlitzten Injektionsstelle (52) und am entgegengesetzten Ende mit einem Fluidauslaß (74) versehen ist, sowie einen rohrförmigen Arm (76) hat, der von dem Gehäuse vorsteht, und wobei das Kanüleneinführelement (82) aufweist: eine Kanüle (98, 104), die eine Fluiddurchflußbahn darin definiert, wobei die Kanüle einen distalen Endbereich zum Durchdringen der Injektionsstelle (52) hat und in dem distalen Endbereich mindestens eine Öffnung hat, durch die Fluid zu oder von der Fluiddurchflußbahn strömen kann, und eine Schutzabschirmung (80), die die Kanüle mindestens teilweise umgibt und um einen vorbestimmten Abstand davon beabstandet ist, wobei das Abschirmung (80) eine bogenförmige Wand (164) hat, die die Kanüle (98, 104) teilweise umgibt und davon beabstandet ist, wobei die bogenförmige Wand eine offene Seite (174) definiert, die Abschirmung über dem Verbindergehäuse (70) in Eingriff bringbar ist, wenn die Kanüle (98, 104) in die Injektionsstelle (52) eingeführt wird, wobei der Verbinderarm (76) in der offenen Seite (174) aufgenommen wird, gekennzeichnet durch mindestens einen bogenförmigen Arm (170), der sich von der bogenförmigen Wand (164) über einen Bereich der offenen Seite (174) erstreckt, wobei der Arm allgemein umfangsmäßig um den distalen Endbereich der Kanüle (98) verläuft, die Abschirmung (80) und der Verbinder relativ drehbar sind, um den bogenförmigen Arm (170) der Abschirmung unter dem Verbinderarm (76) bajonettartig in Eingriff zu bringen, um das Kanüleneinführelement an dem Verbinder zu arretieren, und die Kanüle einen verjüngten distalen Endbereich hat, der zu einem stumpfen distalen Ende führt.

2. Anordnung nach Anspruch 1, wobei der Abschirmungsarm (170) einen Widerhaken (172) hat, über den der Verbinderarm (76) während der relativen Drehung hinweggeht.

3. Anordnung nach Anspruch 1 oder 2, wobei die Schutzabschirmung (80) und der Verbinder mindestens eine Eingriffszone (A1, A2; B1, B2; C1, C2; D1, D2) haben, die eine Ausfluchtung des Kanüleneinführelements (82) mit der Injektionsstelle (52) herstellt.

4. Anordnung nach Anspruch 3, die eine Eingriffszone (A1, A2) zwischen dem Verbinderarm (76) und den Wänden (168, 178) aufweist, die einen Spalt (176) zwischen dem bogenförmigen Arm (170) der Abschirmung definiert.

5. Anordnung nach Anspruch 3 oder 4, die eine Eingriffszone (B1, B2; C1, C2) zwischen dem Inneren der Abschirmung (80) und dem Verbindergehäuse (70) aufweist.

6. Anordnung nach Anspruch 5, die beabstandete Eingriffszonen (B1, B2; C1, C2) zwischen dem Inneren der Abschirmung (80) und dem Verbindergehäuse (70) aufweist.

7. Anordnung nach einem der vorhergehenden Ansprüche, wobei sich die Abschirmung (80) über das stumpfe distale Ende der Kanüle (98, 104) hinaus erstreckt.

8. Anordnung nach einem der vorhergehenden Ansprüche, wobei die Kanüle (104) einen distalen Endbereich (116) hat, der an seiner Außenfläche eine Vielzahl von umfangsmäßig beabstandeten langgestreckten Nuten (124) aufweist, die sich in Längsrichtung der Kanüle erstrecken.

9. Anordnung nach einem der vorhergehenden Ansprüche, wobei die Kanüle (98, 104) eine matte Oberflächenbeschaffenheit hat, um Reibung an der Kanülen/Septum-Grenzfläche zu verringern.

10. Anordnung nach einem der vorhergehenden Ansprüche, wobei der Verbinder ein T-Verbinder ist und die Abschirmung einen weiteren bogenförmigen Arm (170b) hat, um mit einem Arm des Verbinders bajonettartig in Eingriff zu gelangen.

11. Anordnung nach Anspruch 10, wobei der weitere bogenförmige Arm (170b) einen Widerhaken (172b) hat.

## Revendications

1. Ensemble de connecteur en Y ou en T comportant un élément d'insertion de canule, dans lequel le connecteur comprend un boîtier (70) muni à une extrémité d'un site d'injection pré-incisé (52) et à l'extrémité opposée d'une sortie de fluide (74), et un bras tubulaire (76) dépassant du boîtier, et l'élément d'insertion de canule (82) comprend une canule (98, 104) qui y délimite un passage d'écoulement de fluide, la canule présentant une région d'extrémité distale pour pénétrer dans le site d'injection (52) et présentant au moins une ouverture dans la région d'extrémité distale à travers laquelle le fluide peut s'écouler vers le passage d'écoulement de fluide ou à partir de celui-ci, et une gaine protectrice (80) entourant au moins partiellement la canule et étant espacée d'une distance prédéterminée de celle-ci, la gaine (80) présentant une paroi courbe (164) entourant partiellement la canule (98, 104) et étant espacée de celle-ci, la paroi courbe délimitant un côté ouvert (174), la gaine pouvant s'engager sur le boîtier (70) de connecteur lorsque la canule (98, 104) est insérée dans le site d'injection (52), le bras de connecteur (76) étant reçu dans le côté ouvert (174), caractérisé par au moins un bras courbe (170) s'étendant à partir de ladite paroi courbe (164) le long d'une partie dudit côté ouvert (174), ledit bras s'étendant de façon globalement circonférentielle autour de la région d'extrémité distale de la canule (98), la gaine (80) et le connecteur pouvant tourner l'une par rapport à l'autre pour s'engager dans le bras courbe (170) de gaine sous le bras de connecteur (76) en baïonnette afin de bloquer l'élément d'insertion de canule sur le connecteur, et la canule présentant une région d'extrémité distale conique terminée par une extrémité distale arrondie.

2. Ensemble selon la revendication 1, dans lequel le bras (170) de gaine présente une barbe (172) au-dessus de laquelle passe le bras de connecteur (76) pendant ladite rotation relative.

3. Ensemble selon la revendication 1 et 2, dans lequel la gaine protectrice (80) et le connecteur présentent au moins une zone d'engagement (A1, A2 ; B1, B2 ; C1, C2 ; D1, D2) établissant l'alignement de l'élément d'insertion de canule (82) avec le site d'injection (52).

4. Ensemble selon la revendication 3, comprenant une zone d'engagement (A1, A2) entre le bras de connecteur (76) et les parois (168, 178) délimitant un intervalle (176) entre le bras courbe (170) de gaine.

5. Ensemble selon la revendication 3 ou 4, comprenant une zone d'engagement (B1, B2 ; C1, C2) entre l'intérieur de la gaine (80) et le boîtier de connecteur (70).

6. Ensemble selon la revendication 5, comprenant des zones espacées d'engagement (B1, B2 ; C1, C2) entre l'intérieur de la gaine (80) et le boîtier de connecteur (70).

7. Ensemble selon l'une quelconque des revendications précédentes, dans lequel la gaine (80) s'étend audelà de l'extrémité distale arrondie de la canule (98, 104).

8. Ensemble selon l'une quelconque des revendications précédentes, dans lequel la canule (104) présente une partie d'extrémité distale (116) munie sur sa surface extérieure de plusieurs gorges allongées circonférentiellement espacées (124) s'étendant le long de la canule.

9. Ensemble selon l'une quelconque des revendications précédentes, dans lequel la canule (98, 104) pré-sente un fini mat afin de réduire le frottement à l'interface canule/septum.

10. Ensemble selon l'une quelconque des revendications précédentes, dans lequel le connecteur est un connecteur en T et la gaine présente un bras courbe supplémentaire (170b) pour s'engager en baïonnette dans un bras du connecteur.

11. Ensemble selon la revendication 10, dans lequel ledit bras courbe supplémentaire (170b) présente une barbe (172b).
